(19) Europäisches Patentamt
European Patent Office
Office européen des brevets

(11) **EP 3 239 272 B1**

(12) **EUROPEAN PATENT SPECIFICATION**

(45) Date of publication and mention
of the grant of the patent:
**17.07.2019 Bulletin 2019/29**

(51) Int Cl.:
***C09K 11/66*** *(2006.01)* ***H01L 31/00*** *(2006.01)*

(21) Application number: **17167441.9**

(22) Date of filing: **21.04.2017**

(54) **LIGHT ABSORPTION MATERIAL AND SOLAR CELL USING THE SAME**

LICHTABSORPTIONSMATERIAL UND SOLARZELLE DAMIT

MATÉRIAU D'ABSORPTION DE LUMIÈRE ET CELLULE SOLAIRE L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2016 JP 2016087108**
**19.12.2016 JP 2016245613**

(43) Date of publication of application:
**01.11.2017 Bulletin 2017/44**

(73) Proprietor: **Panasonic Intellectual Property Management Co., Ltd.**
**Osaka-shi, Osaka 540-6207 (JP)**

(72) Inventors:
• **SUZUKA, Michio**
**Osaka-shi, Osaka 540-6207 (JP)**
• **UCHIDA, Ryuusuke**
**Osaka-shi, Osaka 540-6207 (JP)**
• **YOKOYAMA, Tomoyasu**
**Osaka-shi, Osaka 540-6207 (JP)**

(74) Representative: **Grünecker Patent- und Rechtsanwälte**
**PartG mbB**
**Leopoldstraße 4**
**80802 München (DE)**

(56) References cited:
• **GILES E. EPERON ET AL: "Formamidinium lead trihalide: a broadly tunable perovskite for efficient planar heterojunction solar cells", ENERGY & ENVIRONMENTAL SCIENCE, vol. 7, no. 3, 1 January 2014 (2014-01-01) , pages 982-988, XP055234624, ISSN: 1754-5692, DOI: 10.1039/c3ee43822h**
• **TECK MING KOH ET AL: "Formamidinium-Containing Metal-Halide: An Alternative Material for Near-IR Absorption Perovskite Solar Cells", JOURNAL OF PHYSICAL CHEMISTRY C, vol. 118, no. 30, 31 July 2014 (2014-07-31), pages 16458-16462, XP055343492, US ISSN: 1932-7447, DOI: 10.1021/jp411112k**
• **SARAH WOZNY ET AL: "Controlled Humidity Study on the Formation of Higher Efficiency Formamidinium Lead Triiodide-Based Solar Cells", CHEMISTRY OF MATERIALS, vol. 27, no. 13, 14 July 2015 (2015-07-14) , pages 4814-4820, XP055407869, US ISSN: 0897-4756, DOI: 10.1021/acs.chemmater.5b01691**
• **ANDREAS BINEK ET AL: "Stabilization of the Trigonal High-Temperature Phase of Formamidinium Lead Iodide", JOURNAL OF PHYSICAL CHEMISTRY LETTERS, vol. 6, no. 7, 2 April 2015 (2015-04-02), pages 1249-1253, XP055408042, US ISSN: 1948-7185, DOI: 10.1021/acs.jpclett.5b00380**

**Description**

BACKGROUND

1. Technical Field

The present disclosure relates to a light absorption material and a solar cell using the same.

2. Description of the Related Art

In recent years, research and development of perovskite solar cells has been performed. In the perovskite solar cells, perovskite crystals denoted by $ABX_3$ (A represents a monovalent cation, B represents a divalent cation, and X represents a halogen anion) and structures analogous thereto (hereafter referred to as "perovskite compounds") are used as light absorption materials.

Jeong-Hyeok Im and four colleagues, "Nature Nanotechnology" (U.S.), November, 2014, Vol. 9, p. 927-932 discloses that a perovskite compound represented by $CH_3NH_3PbI_3$ (hereafter also abbreviated as "$MAPbI_3$") is used as a light absorption material of a perovskite solar cell. Also, the above-described literature discloses a perovskite compound represented by $(NH_2)_2CHPbI_3$ (hereafter also abbreviated as "$FAPbI_3$").

SUMMARY

It is desired to enhance the conversion efficiency of a perovskite solar cell.

In one general aspect, the techniques disclosed here feature a light absorption material according to claim 1 comprising: a compound having a perovskite crystal structure represented by $ABX_3$ where the A site contains $(NH_2)_2CH^+$, the B site contains $Pb^{2+}$, and the X site contains $I^-$. A ratio of the number of atoms of I to the number of atoms of Pb measured by an X-ray photoelectron spectroscopy is 2.7 or less, or a ratio of the number of atoms of I to the number of atoms of Pb measured by a Rutherford backscattering spectroscopy is 2.9 or less.

It should be noted that general or specific embodiments may be implemented as a system, a method, an integrated circuit, a computer program, a storage medium, or any selective combination thereof.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

BRIEF DESCRIPTION OF THE DRAWINGS

Fig. 1 is a diagram showing the relationship between the fluorescence lifetime and the conversion efficiency of a perovskite solar cell;
Fig. 2A is a schematic diagram illustrating an example of a method for manufacturing a perovskite compound according to the present disclosure;
Fig. 2B is a schematic diagram illustrating an example of a method for manufacturing a perovskite compound according to the present disclosure;
Fig. 2C is a schematic diagram illustrating an example of a method for manufacturing a perovskite compound according to the present disclosure;
Fig. 3A is a magnified photograph showing an example of the perovskite compound according to the present disclosure;
Fig. 3B is a magnified photograph showing another example of the perovskite compound according to the present disclosure;
Fig. 4A is a diagram showing XPS spectra of perovskite compounds of example 2 and comparative example 2 and is a diagram showing spectra around the binding energy corresponding to C1s;
Fig. 4B is a diagram showing XPS spectra of perovskite compounds of example 2 and comparative example 2 and is a diagram showing spectra around the binding energy corresponding to N1s;
Fig. 4C is a diagram showing XPS spectra of perovskite compounds of example 2 and comparative example 2 and is a diagram showing spectra around the binding energy corresponding to Pb4f;
Fig. 4D is a diagram showing XPS spectra of perovskite compounds of example 2 and comparative example 2 and is a diagram showing spectra around the binding energy corresponding to I3d5;

Fig. 5A is a diagram showing an X-ray diffraction pattern of a perovskite compound of example 1;

Fig. 5B is a diagram showing an X-ray diffraction pattern of a perovskite compound of comparative example 1;

Fig. 6 is a diagram showing fluorescence spectra of the perovskite compounds of example 1 and comparative example 1;

Fig. 7 is a diagram showing fluorescence decay curves of the perovskite compounds of example 1 and comparative example 1;

Fig. 8 is a diagram showing absorption spectra of the perovskite compounds of example 1 and comparative example 1;

Fig. 9 is a schematic sectional view showing an example of a solar cell according to the present disclosure;

Fig. 10 is a schematic sectional view showing another example of the solar cell according to the present disclosure;

Fig. 11 is a schematic sectional view showing another example of the solar cell according to the present disclosure;

Fig. 12 is a schematic sectional view showing another example of the solar cell according to the present disclosure;

Fig. 13A is a diagram showing XRD patterns of the compounds of example 1 and comparative example 1;

Fig. 13B is a diagram showing XRD patterns of the compounds of example 1 and comparative example 1; and

Fig. 14 is a diagram showing the IPCE measurement results of example 11 and comparative example 4.

## DETAILED DESCRIPTION

Embodiments

The present disclosure is based on the findings described below.

It is known that the performance of a light absorption material for a solar cell is determined by the band gap thereof. There are detailed descriptions in William Shockley and colleague, "Journal of Applied Physics" (U.S.), March, 1961, Vol. 32, No. 3, p. 510-519. The limit of this conversion efficiency is known as the Shockley-Queisser limit and the theoretical efficiency is at maximum when the band gap is 1.4 eV. In the case where the band gap is larger than 1.4 eV, a high voltage is obtained, but a current value decreases due to a decrease in the wavelength of light absorption. Conversely, in the case where the band gap is smaller than 1.4 eV, the current increases due to an increase in the wavelength of light absorption, but the open circuit voltage decreases.

However, band gaps of perovskite compounds in the related art deviate from 1.4 eV, at which the theoretical efficiency is at maximum, to a great extent. For example, the band gap of the above-described $MAPbI_3$ perovskite compound is 1.59 eV, and the band gap of the $FAPbI_3$ perovskite compound is 1.49 eV. Consequently, a perovskite compound having a band gap of 1.4 eV or closer to 1.4 eV has been required. In the case where such a perovskite compound is used as the light absorption material for a solar cell, a solar cell having a conversion efficiency higher than that of a conventional one can be realized.

In general, $FAPbI_3$ has a trigonal structure. As reported in Giles E Eperon and five colleagues, "Energy & Environmental Science" (U.K.), 2014, Vol. 7, No. 3, p. 982-988, the band gap of $FAPbI_3$ is, for example, 1.48 eV. Regarding $FAPbI_3$, in the perovskite crystal structure denoted by $ABX_3$, $FA^+$ (formamidinium cation) occupies the A site, $Pb^{2+}$ occupies the B site, and $I^-$ occupies the X site. Some of the X sites may be substituted with bromine (Br) or the like.

According to the explanation in, for example, p. 1487 to p. 1488 of Shuping Pang and nine colleagues, "Chemistry of Materials" (U.S.), January, 2014, Vol. 26, p. 1485-1491, a crystal of $FAPbI_3$ including $FA^+$ cannot have a cubic structure, and the structure near to $FA^+$ is distorted.

Further, Marina R. Filip and three colleagues, "Nature Communications" (U.S.), December, 2014, Vol. 5, 5757 shows systematic calculation results with respect to perovskite compounds in which the A site of $FAPbI_2$ is substituted with various cations. The authors paid attention to angles (Pb-I-Pb bond angles) between $PbI_6$ octahedrons, and reported that in the case where $PbI_6$ octahedrons were ideally arranged, i.e., $PbI_6$ octahedrons were arranged without distortion, a small band gap was obtained, and in the case where the $PbI_6$ octahedrons were zigzag arranged, a large band gap was obtained. Also, it is shown that the structure of $FAPbI_3$ is distorted to some extent, and the structure is not perfectly symmetric.

As reported by Marina R. Filip et al. and Shuping Pang et al., $FAPbI_3$ has a trigonal structure basically, and the $PbI_6$ octahedral structure thereof is distorted. It is considered that the distortion of the $PbI_6$ octahedral structure causes $FAPbI_3$ to have a large band gap of about 1.49 eV.

In consideration of these studies, the present inventor performed investigations and, as a result, found a new $FAPbI_3$ perovskite compound having small distortion between octahedrons and having a band gap smaller than that of a conventional one.

The outline of an embodiment according to the present disclosure is described below.

Item 1

A light absorption material comprising:

a compound having a perovskite crystal structure represented by $ABX_3$ where the A site contains $(NH_2)_2CH^+$, the B site contains $Pb^{2+}$, and the X site contains $I^-$, wherein
a ratio of the number of atoms of I to the number of atoms of Pb measured by an X-ray photoelectron spectroscopy is 2.7 or less, or
a ratio of the number of atoms of I to the number of atoms of Pb measured by a Rutherford backscattering spectroscopy is 2.9 or less.

Item 2

The light absorption material according to Item 1, wherein the ratio of the number of atoms of I to the number of atoms of Pb measured by an X-ray photoelectron spectroscopy is 1.8 or more.

Item 3

The light absorption material according to Item 2, wherein the ratio of the number of atoms of I to the number of atoms of Pb measured by an X-ray photoelectron spectroscopy is 2.1 or more.

Item 4

The light absorption material according to Item 1, wherein the ratio of the number of atoms of I to the number of atoms of Pb measured by a Rutherford backscattering spectroscopy is 2.0 or more.

Item 5

The light absorption material according to Item 4, wherein the ratio of the number of atoms of I to the number of atoms of Pb measured by a Rutherford backscattering spectroscopy is 2.3 or more.

Item 6

The light absorption material according to Item 1, wherein the fluorescence spectrum of the compound has a peak at 880 nm or more and 905 nm or less.

Item 7

The light absorption material according to Item 1, wherein
an X-ray diffraction pattern of the compound has a first peak at $13.9° \leq 2\theta \leq 14.1°$, a second peak at $23.5° \leq 2\theta \leq 24.5°$, and a third peak at $27° \leq 2\theta \leq 29°$,
an intensity of the second peak is equal to or smaller than 40% of an intensity of the first peak, and
an intensity of the third peak is greater than an intensity of the first peak.

Item 8

The light absorption material according to Item 7, wherein the X-ray diffraction pattern of the compound has a fourth peak at $19.65° \leq 2\theta \leq 19.71°$.

Item 9

The light absorption material according to Item 7 or 8, wherein the X-ray diffraction pattern of the compound has a fifth peak at $39.98° \leq 2\theta \leq 40.10°$.

Item 10

A solar cell comprising:

a first electrode having electrical conductivity;
a second electrode having electrical conductivity; and
a light absorption layer between the first electrode and the second electrode, the light absorption layer comprising the light absorption material according to any of Items 1 to 9 and converting incident light into a charge.

Composition and crystal structure of perovskite compound

Composition

The light absorption material according to an embodiment of the present disclosure primarily contains a perovskite compound having a perovskite crystal structure denoted by $ABX_3$. Here, $(NH_2)_2CH^+$ occupies the A site, $Pb^{2+}$ occupies the B site, and $I^-$ occupies the X site. In the perovskite compound according to an embodiment of the present disclosure, a ratio of the number of atoms of I to the number of atoms of Pb is less than 3. In other words, x > 0 is satisfied when an element ratio of Pb is specified as 1 and an element ratio of I to Pb is specified as $3 \times (1 - x)$. In the present specification, the value of x is referred to as a deficiency rate of I (iodine) relative to Pb (hereafter abbreviated as "iodine deficiency rate").

The iodine deficiency rate x is determined by performing composition analysis of a perovskite compound. For example, the content of each element may be determined by composition analysis, and the iodine deficiency rate x may be calculated from the content of I relative to the content of Pb. X-ray photoelectron spectroscopy (XPS), Rutherford backscattering spectroscopy (RBS), nuclear reaction analysis (NRA), and the like may be performed as the composition analysis. The analytical result (ratio of element) may differ according to the composition analysis method. This is because the sensitivity of each composition analysis method may differ according to measurement area, measurement depth, and the state of an element. As an example, regarding the composition analysis by the XPS measurement, the element ratio $3 \times (1 - x)$ of I to Pb is, for example, 1.8 or more and 2.7 or less, and the iodine deficiency rate x is, for example, 0.1 or more and 0.4 or less.

The light absorption material according to the present embodiment has to primarily contain the above-described perovskite compound and may contain impurities. The proportion of the perovskite compound in the light absorption material may be, for example, 90 percent by weight or more. The light absorption material according to the present embodiment may further contain other compounds different from the perovskite compound.

Next, an example of the analytical result of the perovskite compound according to the present disclosure will be described.

X-ray diffraction pattern

Regarding X-ray diffraction by using CuKα rays, the X-ray diffraction (XRD) pattern of the perovskite compound according to the present embodiment has peaks at 2θ of, for example, 13.9° to 14.1°, 19° to 21°, 23.5° to 24.5°, 27° to 29°, 30.5° to 32.5°, 33.5° to 35.5°, and 39° to 41°. In the case where the light absorption material according to the present disclosure is not a mixture, each of the peaks appears as a single peak and a plurality of peaks do not appear in each of the above-described ranges. In the case where the light absorption material according to the present disclosure is not a mixture, a peak other than those described above does not appear at a diffraction angle of 41° or less.

In the present specification, among the above-described X-ray diffraction pattern peaks, the peak located at $13.9° \leq 2\theta \leq 14.1°$ is referred to as a "first peak", the peak located at $23.5° \leq 2\theta \leq 24.5°$ is referred to as a "second peak" and the peak located at $27° \leq 2\theta \leq 29°$ is referred to as a "third peak". The first peak is assigned to the (100) planes, the second peak is assigned to the (111) planes, and the third peak is assigned to the (200) planes. The peak located at 19° to 21° and assigned to the (110) planes is referred to as a "fourth peak", and the peak located at 39° to 41° and assigned to the (220) planes is referred to as a "fifth peak". The fourth peak may be located at, for example, $19.65° \leq 2\theta \leq 19.71°$, and the fifth peak may be located at, for example $39.98° \leq 2\theta \leq 40.10°$.

The X-ray diffraction pattern of the perovskite compound according to the present embodiment is different from that of $FAPbI_3$ in the related art in the following points. First, regarding the perovskite compound according to the present embodiment, the intensity ratio $I_{(111)}/I_{(100)}$ of the second peak to the first peak is, for example, more than 0% and 40% or less. Regarding $FAPbI_3$ in the related art, the intensity ratio $I_{(111)}/I_{(100)}$ of the second peak to the first peak is, for example, 49%. That is, the intensity ratio $I_{(111)}/I_{(100)}$ of the second peak to the first peak of the perovskite compound according to the present embodiment is smaller than that of the $FAPbI_3$ in the related art. Second, regarding the perovskite compound according to the present embodiment, the intensity of the third peak is larger than the intensity of the first peak. On the other hand, regarding $FAPbI_3$ in the related art, the intensity of the third peak is smaller than the intensity of the first peak.

The present inventor theoretically calculated the X-ray diffraction pattern of $FAPbI_3$ while changing the element ratios of iodine, lead, and the like. As a result, it was found that the above-described features of the X-ray diffraction pattern of the perovskite compound according to the present embodiment were features of an X-ray diffraction pattern that appeared in a case where iodine deficiency was included in a perovskite compound. Therefore, the XRD measurement results indicate that the perovskite compound according to the present embodiment includes more iodine deficiencies than the $FAPbI_3$ perovskite compound in the related art does.

Fluorescence spectrum

The peak of the fluorescence spectrum of the perovskite compound according to the present embodiment is located at a wavelength longer than that of the $FAPbI_3$ in the related art. The fluorescence spectrum of $FAPbI_3$, in the related art, excited by visible light (for example, 532 nm) has a peak at 830 nm. Meanwhile, the fluorescence spectrum of the perovskite compound according to the present embodiment has a peak at, for example, 880 nm or more and 905 nm or less. The perovskite compound according to the present embodiment absorbs light with a long wavelength (for example, light with a wavelength of 830 nm or more). The above-described fluorescence spectrum indicates that the feature is not based on an impurity level but based on the presence of a high electron density distribution in a conduction band and a valence band in the same manner as a so-called direct transition semiconductor.

Physical properties of perovskite compound

The perovskite compound according to the present embodiment can have the following physical properties useful for the light absorption material for the solar cell.

Band gap

The perovskite compound according to the present embodiment can have a band gap close to 1.4 eV compared with the band gap (1.49 eV) of $FAPbI_3$ in the related art. The band gap of the perovskite compound according to the present embodiment is preferably about 1.4 eV, for example, 1.35 eV or more and less than 1.45 eV.

The band gap of the perovskite compound is calculated on the basis of, for example, the absorbance of the perovskite compound.

It is considered that the perovskite compound according to the present embodiment has a band gap smaller than that of a conventional one because of the following reason.

As described above, in the $FAPbI_3$ perovskite crystal in the related art, a plurality of $PbI_6$ octahedrons are arranged in a zigzag due to the sizes of constituent ions, and it is difficult for the $PbI_6$ octahedrons to be linearly arranged. Meanwhile, regarding the perovskite compound according to the present embodiment, in the perovskite crystal structure, some of the iodine sites constituting the $PbI_6$ octahedron are deficient in iodine. The proportion of such iodine-deficient sites is higher than that in $FAPbI_3$ in the related art. It is considered that distortion of the structure due to a large $FA^+$ is relaxed by the iodine-deficient sites, symmetry is improved, and the arrangement of $PbI_6$ octahedrons becomes linear more than a conventional one is. As a result, the band gap is decreased to 1.4 eV at which the light absorption material for the solar cell can realize the highest efficiency.

Fluorescence lifetime

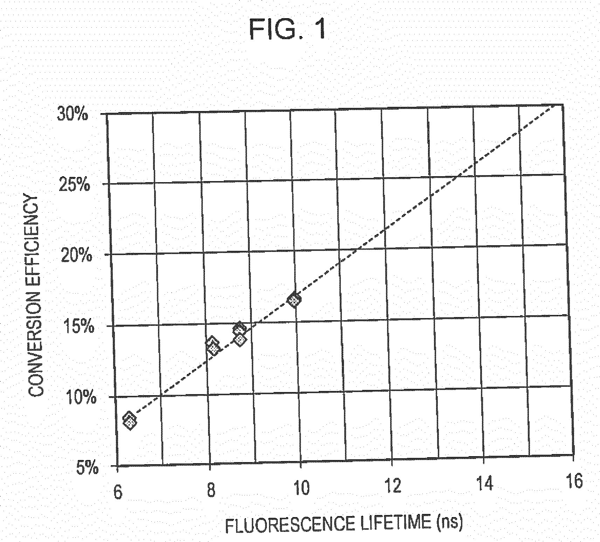

The "fluorescence lifetime" refers to the lifetime (decay rate constant) of fluorescence generated by recombination of electrons in the conduction band and holes in the valence band after charge separation. Fig. 1 was obtained based on investigations by the present inventor. Fig. 1 is a diagram showing the relationship between the fluorescence lifetime and the conversion efficiency of a perovskite compounds fabricated referring to the related arts. As shown in Fig. 1, the conversion efficiency increases as the fluorescence lifetime increases. This is because the recombination rate decreases as the fluorescence lifetime increases and, as a result, electrons and holes that can be output increase in number,

The fluorescence lifetime is measured at a wavelength at which the fluorescence intensity is the highest, where excitation is performed at a wavelength of 532 nm. The measurement temperature is 25°C. Here, quadratic linear approximation is performed from the time at which the intensity count is the highest, and the decay rate constant of the component having a long lifetime is calculated as the "fluorescence lifetime".

The fluorescence lifetime of the $FAPbI_3$ perovskite compound in the related art is, for example, about 5 ns to 10 ns. The fluorescence lifetime of the perovskite compound according to the present embodiment is longer than that of a conventional one and is, for example, more than 10 ns, and desirably 15 ns or more. Consequently, the conversion efficiency of the solar cell can be further enhanced.

Method for manufacturing light absorption material

Next, an example of a method for manufacturing the perovskite compound according to the present embodiment will be described with reference to the drawings. Here, a method for growing a crystal in a liquid phase will be described as an example, but the manufacturing method according to the present embodiment is not limited to this.

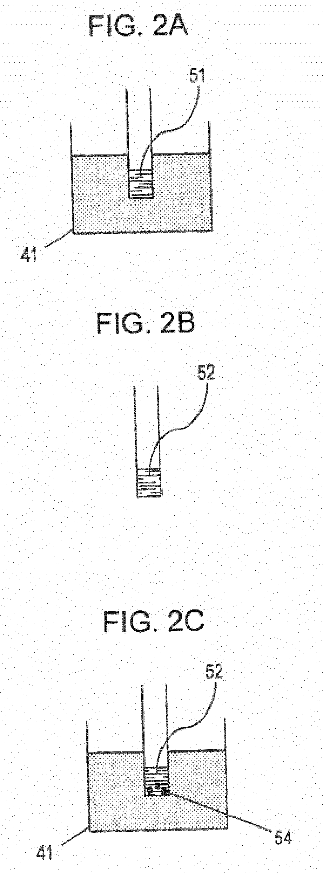

As shown in Fig. 2A, $PbI_2$ and FAI in the same molar amount as $PbI_2$ are added to γ-butyrolactone (γ-BL). The molar amount of each of $PbI_2$ and FAI is, for example, 1 M. A yellow solution (first solution) 51 is produced by performing dissolution in an oil bath 41 heated to, for example, 40°C or higher and 80°C or lower (80°C, here).

The first solution 51 is cooled to room temperature once and, thereafter, as shown in Fig. 2B, pure water is mixed into the first solution 51 while agitation is sufficiently performed so as to produce a second solution 52. The amount of the pure water added may be, for example, 0.1 percent by volume or more and 1.0 percent by volume or less (0.7 percent by volume, here) on the basis of a volume ratio relative to the first solution 51. The resulting second solution 52 is left to stand (store) at room temperature.

As shown in Fig. 2C, the second solution 52 is put into a test tube and is left to stand in the oil bath 41 heated to 90°C or higher and 130°C or lower (110°C, here). Consequently, black crystals 54 are deposited inside the liquid. The standing time (deposition time) Td in the oil bath 41 may be, for example, 0.5 hours or more and 5 hours or less, and desirably 1 hour or more and 3 hours or less. Thereafter, the crystals 54 are sufficiently washed with acetone. In this manner, a perovskite compound ($FAPbI_3$ crystal) can be produced.

The resulting $FAPbI_3$ crystal has iodine deficiencies. The amount of iodine deficiencies can be adjusted by process conditions. For example, the amount of iodine deficiencies can be changed by the storing time Ts of the second solution 52. As the storing time Ts increases, the period of exposure of the second solution 52 to air (oxygen) increases and, thereby, iodine ions ($I^-$) in the second solution 52 react with oxygen so as to isolate iodine ($I_2$) easily (refer to formulae below). Consequently, the amount of iodine deficiencies can increase.

$$2I^- \rightarrow I_2 + 2e^-$$

$$O_2 + 4e^- + 4H_2O \rightarrow 4OH^-$$

The storing time Ts may be, for example, 1 hour or more and 30 days or less, although it cannot be generalized because the storing time Ts also changes with other process conditions.

The amount of iodine deficiencies can also be adjusted by adjusting the amount of the water added to the first solution 51. If the amount of the water added increases, the amount of iodine deficiencies tends to increase. However, as described above, the amount of the water added to the first solution 51 is adjusted within the range of 0.1 percent by volume or more and 1.0 percent by volume or less. If the amount of the water added is excessively large (for example, more than 1.0 percent by volume), deposition of crystals does not occur in some cases. If the amount of the water added is excessively small (for example, less than 0.1 percent by volume), iodine deficiencies are not caused or the amount of iodine deficiencies may become smaller than a predetermined amount in some cases.

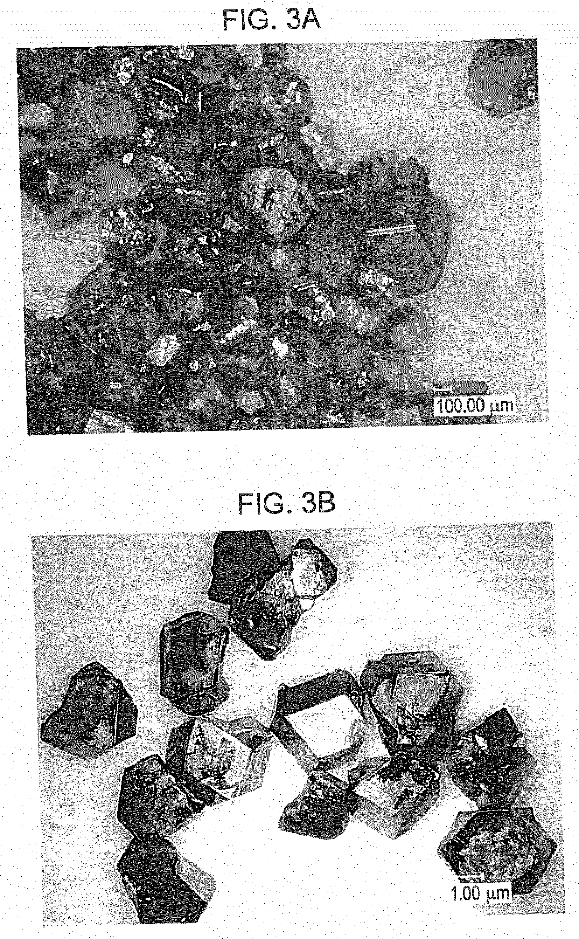

Fig. 3A is a magnified photograph of the perovskite compound produced while the deposition time Td was set to be 1 hour. Fig. 3B is a magnified photograph of the perovskite compound produced while the deposition time Td was set to be 3 hours. As the deposition time Td increased, the crystal size increased and, therefore, it is clear that the crystal size can be adjusted by the deposition time Td.

Among three Hansen solubility parameters (HSPs) of a solvent used for the first solution 51 and the second solution 52, $\sigma_h$ (energy due to intermolecular hydrogen bonding) and $\sigma_p$ (energy due to intermolecular dipole-dipole interaction) may be within the following respective ranges.

$$4 < \sigma_h < 14 \quad (1)$$

$$10 < \sigma_p < 22 \quad (2)$$

Alternatively, $\sigma_h$ and $\sigma_p$ may be within the following respective ranges.

$$5 < \sigma_h < 8 \quad (3)$$

$$13 < \sigma_p < 18 \quad (4)$$

Regarding the solvent having HSPs satisfying the above-described ranges, the strength of interaction between a lead halide or alkylammonium halide, which is a precursor of the perovskite compound, and the solvent molecule is within an appropriate range. Therefore, dissolution of the precursor molecule into the solvent and deposition of the perovskite compound by inverse temperature crystallization (ITC) can be made compatible with each other. ITC refers to a phenomenon in which the solubility of a solute into a solvent decreases in accordance with an increase in temperature and a crystal is deposited.

Specific examples of such solvents include lactone-based solvents. Examples of lactone-based solvents include γ-butyrolactone, caprolactone, γ-valerolactone, γ-hexanolactone, γ-nonalactone, and derivatives thereof. A plurality of lactone-based solvents may be used in combination so that HSPs satisfy the above-described ranges.

Lactone-based solvents having an alkyl group in a side chain may be used. Examples of lactone-based solvents having an alkyl group in a side chain include γ-valerolactone (side chain: -CH$_3$), γ-hexanolactone (side chain: -CH$_2$CH$_3$), and γ-nonalactone (side chain: -(CH$_2$)$_4$CH$_3$). According to investigations of the present inventor, it was ascertained that the deposition temperature of the perovskite compound decreased as the length of an alkyl group of a side chain of the lactone-based solvent increased. Specifically, the deposition temperature of γ-butyrolactone having no side chain was 120°C, that of γ-valerolactone was 80°C, that of γ-hexanolactone was 70°C, and that of γ-nonalactone was 30°C. A low crystal deposition temperature is industrially useful because energy consumption during production can be reduced.

Examples and comparative examples

In examples and comparative examples, perovskite compounds (hereafter simply abbreviated as "compounds") were produced and the physical properties were evaluated. The production methods, the evaluation methods, and the results thereof will be described.

Production of compounds of examples and comparative examples

Examples 1 to 10

Compounds of the examples were produced by the method described above with reference to Fig. 2A to Fig. 2C. Specifically, a yellow solution (first solution) was produced by adding 1 mol/L of $PbI_2$ (produced by TOKYO KASEI KOGYO CO., LTD.) and 1 mol/L of FAI (produced by TOKYO KASEI KOGYO CO., LTD.) to γ-butyrolactone (γ-BL), and performing dissolution in an oil bath heated to 80°C. The first solution was cooled to room temperature once and, thereafter, 0.7 percent by volume, on a volume ratio basis, of pure water was mixed while agitation was sufficiently performed. The resulting liquid (second solution) was stored at room temperature for a predetermined time (storing time Ts).

The second solution was put into a test tube and was left to stand in the oil bath heated to 110°C. The time of standing (deposition time Td) was set to be 1 hour. Consequently, black crystals were deposited inside the liquid. Thereafter, the crystals were sufficiently washed with acetone so as to produce a compound ($FAPbI_3$ crystal) of the example. In this regard, the storing time Ts of the second solution was changed and, thereby, compounds of example 1 to example 10 were produced. The storing time Ts of each example is shown in Table 1. The compounds of example 1, example 2, and example 5 were produced under the same production condition and, therefore, are assumed to be substantially the same compound. Likewise, the compounds of example 3 and example 4 are assumed to be substantially the same compound. Likewise, the compounds of example 6 and example 7 are assumed to be substantially the same compound. Comparative examples 1 to 3

A dimethyl sulfoxide (DMSO) solution containing $PbI_2$ with a concentration of 1 mol/L and FAI with a concentration of 1 mol/L was prepared. A substrate was coated with the resulting solution by a spin coating method. An electrically conductive glass substrate (produced by Nippon Sheet Glass Co., Ltd.) that is provided with a fluorine-doped $SnO_2$ layer and has a thickness of 1 mm was used as the substrate. The substrate was heat-treated on a hot plate at 150°C so as to produce a compound ($FAPbI_3$ film) of each of comparative example 1 to comparative example 3. In this regard, the compounds of comparative example 1 to comparative example 3 were produced under the same production condition and, therefore, are assumed to be substantially the same compound. Composition analysis

The compound of each of the examples and comparative examples was analyzed. The compounds of examples 1 and 8 to 10 and comparative example 1 were subjected to RBS measurement and NRA measurement. The compounds of examples 2 to 5 and comparative example 2 were subjected to XPS measurement. The compounds of examples 6 and 7 and comparative example 3 were subjected to electron probe microanalyzer (EPMA) measurement.

The measurement results are shown in Table 1. Table 1 shows the element ratio (atomic percent) of each of the compounds and element ratios of I and C normalized in a case where the element ratio of Pb was 1, which are element ratios of I and C to Pb. Also, the results of calculation of the iodine deficiency rates x in a case where the element ratio of I to Pb was $3 \times (1 - x)$ are shown in Table 1.

Table 1

| | Storing time Ts | Analysis method | Element ratio | | | | Element ratio relative to Pb | | | Iodine deficiency rate x |
|---|---|---|---|---|---|---|---|---|---|---|
| | | | Pb | I | C | N | Pb | I | C | |
| Example 1 | 2 hours | RBS/NRA | 14.6 | 41.8 | 21.8 | 21.8 | 1 | 2.86 | 1.49 | 0.05 |
| Comparative example 1 | - | | 15.8 | 47.5 | 14.7 | 22.0 | 1 | 3.01 | 0.93 | |
| Example 2 | 2 hours | XPS | 16 | 41 | 20 | 23 | 1 | 2.56 | 1.25 | 0.15 |
| Example 3 | 4 days | | 19 | 45 | 15 | 21 | 1 | 2.37 | 0.79 | 0.21 |
| Example 4 | 4 days | | 19 | 46 | 14 | 22 | 1 | 2.42 | 0.74 | 0.19 |
| Example 5 | 2 hours | | 16 | 42 | 17 | 23 | 1 | 2.63 | 1.06 | 0.13 |
| Comparative example 2 | - | | 16 | 44 | 15 | 25 | 1 | 2.75 | 0.94 | 0.08 |

(continued)

| | Storing time Ts | Analysis method | Element ratio | | | | Element ratio relative to Pb | | | Iodine deficiency rate x |
|---|---|---|---|---|---|---|---|---|---|---|
| | | | Pb | I | C | N | Pb | I | C | |
| Example 6 | 3 days | EPMA | 20 | 31 | 21 | 28 | 1 | 1.55 | 1.05 | 0.48 |
| Example 7 | 3 days | | 20 | 31 | 20 | 28 | 1 | 1.53 | 1.00 | 0.49 |
| Comparative example 3 | - | | 18 | 45 | 16 | 21 | 1 | 2.5 | 0.89 | 0.11 |
| Example 8 | 1 hour | RBS/NRA | 13.8 | 38.9 | 14.5 | 32.0 | 1 | 2.82 | 1.05 | 0.06 |
| Example 9 | 1.5 hours | | 13.5 | 39.0 | 13.5 | 33.6 | 1 | 2.9 | 1 | 0.03 |
| Example 10 | 2.5 hours | | 14.3 | 40.8 | 14.7 | 29.6 | 1 | 2.86 | 1.03 | 0.05 |

From the results shown in Table 1, it was ascertained that the measurement results of the element ratio differed according to the analysis method but the compounds of the example 1 to example 10 exhibited iodine deficiency rates x higher than the iodine deficiency rates x of comparative examples 1 to 3.

According to the XPS measurement, the element ratios of I to Pb of the compounds of examples 2 to 5 were within the range of 2.3 or more and 2.7 or less and, therefore, were 2.7 or less (iodine deficiency rate x: 0.1 or more) in all cases.

According to the RBS/NRA measurement, the element ratios of I to Pb of the compounds of examples 1 and 8 to 10 were within the range of 2.8 or more and 2.9 or less and, therefore, were 2.9 or less (iodine deficiency rate x: 0.03 or more) in all cases.

The composition ratio can be calculated with high accuracy by the RBS measurement. For example, even in the case where other compounds are accumulated on a perovskite film, the composition of the perovskite film can be analyzed with high accuracy.

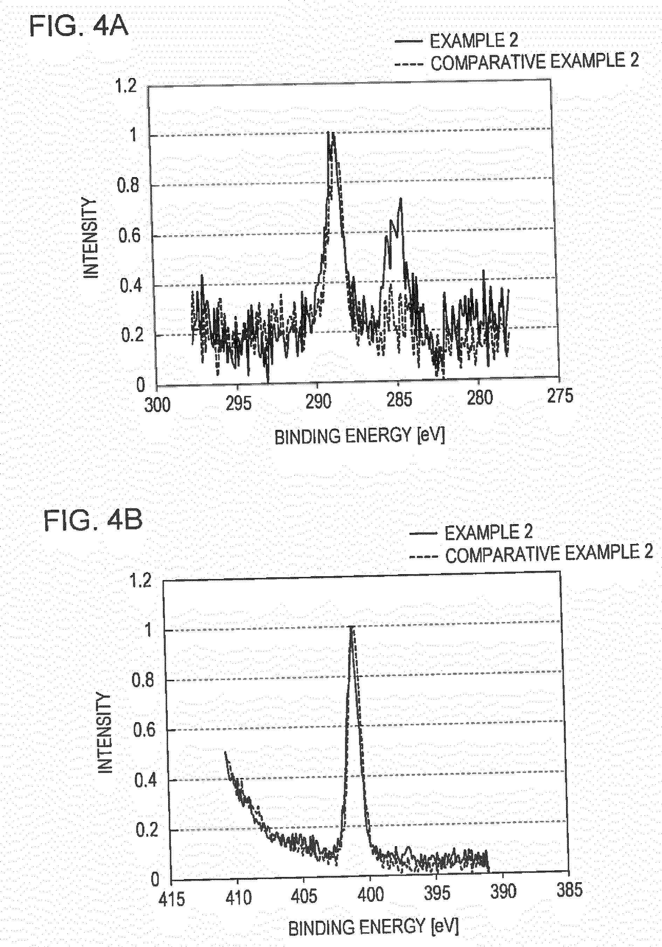

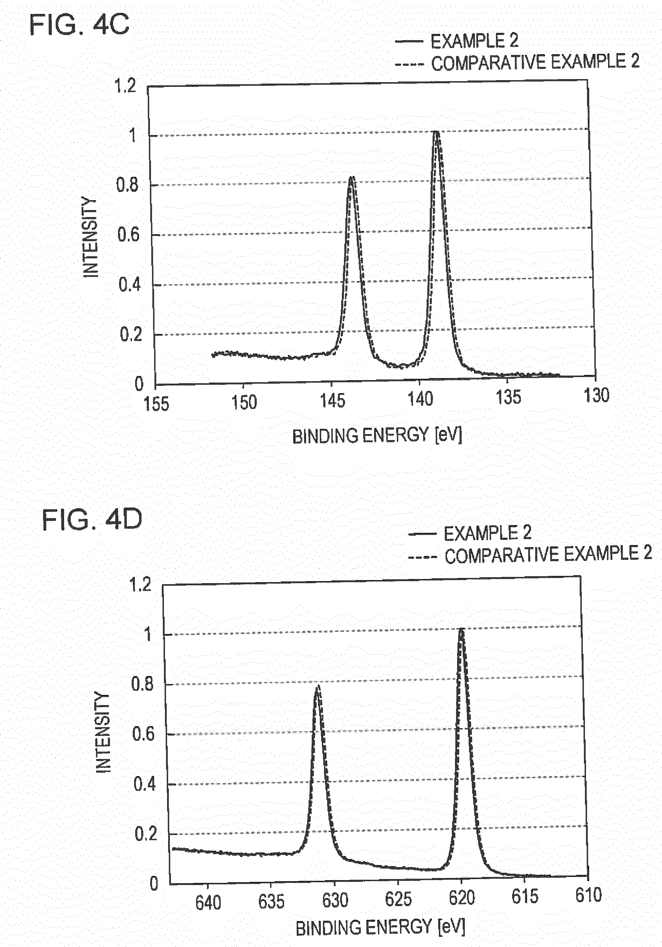

Next, the measurement results of XPS will be described. Fig. 4A to Fig. 4D are diagrams showing XPS spectra of compounds of example 2 and comparative example 2 and show spectra around the binding energy corresponding to each of the C1s orbital, the N1s orbital, the Pb4f orbital, and I3d5 orbital, respectively.

As is clear from results shown in Fig. 4A to Fig. 4D, the spectra corresponding to the N1s orbital, the Pb4f orbital, and I3d5 orbital of example 2 substantially agree with those of comparative example 2. Meanwhile, the spectra corresponding to the C1s orbital are different from each other. Specifically, in example 2, the ratio of the peak intensity of C1s located at 285 to 288 eV relative to the peak intensity of C1s located at 288 to 292 eV is 30% or more, and this ratio is larger than that in comparative example 2. This indicates that there is a difference in the electron state of $FA^+$ between the compound of example 2 and the compound of comparative example 2. In this regard, the XPS spectra in other examples exhibit the same tendency as example 2, although not shown in the drawing.

Crystal structure analysis

The compounds of example 1 to example 5 and comparative example 1 were subjected to the XRD measurement.

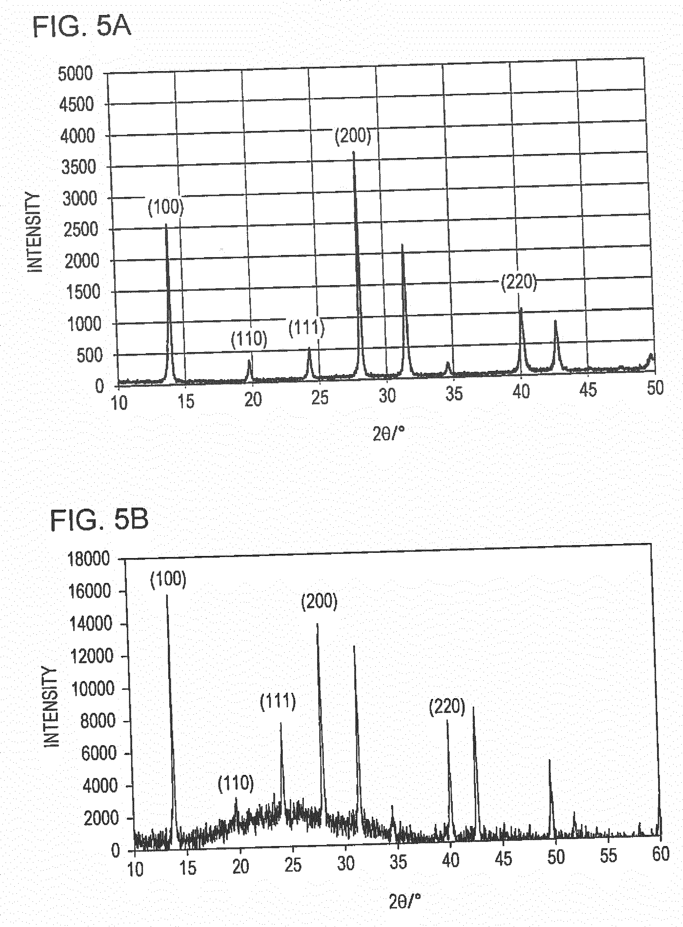

Fig. 5A is a diagram showing the XRD measurement results of the material in example 1, the horizontal axis is for 2θ, and the vertical axis is for the X-ray diffraction intensity. Fig. 5B is a diagram showing the XRD pattern of the compound of comparative example 1. Here, CuKα rays are used as X-rays. The X-ray diffraction patterns of other examples exhibit the same tendency as example 1 and, therefore, are not shown in the drawing.

As shown in Fig. 5A and Fig. 5B, the X-ray diffraction patterns of the compounds of example 1 and comparative example 1 have the first peak that is assigned to the (100) planes and is located at 13.9° ≤ 2θ ≤ 14.1°, the second peak that is assigned to the (111) planes and is located at 23.5° ≤ 2θ ≤ 24.5°, and the third peak that is assigned to the (200) planes and is located at 27° ≤ 2θ ≤ 29°.

The intensity $I_{(100)}$ of the first peak, the intensity $I_{(111)}$ of the second peak, and the intensity $I_{(200)}$ of the third peak of each of the examples and comparative examples are shown in Table 2. Also, the results of calculation of the intensity ratio $I_{(111)}/I_{(100)}$ of the second peak to the first peak and the intensity ratio $I_{(200)}/I_{(100)}$ of the third peak to the first peak are shown in Table 2.

Table 2

| | Peak intensity $I_{(100)}$ $2\theta:13.9\text{-}14.1°$ | Peak intensity $I_{(111)}$ $2\theta:23.5\text{-}24.5°$ | Peak intensity $I_{(200)}$ $2\theta: 27\text{-}29°$ | Intensity ratio $I_{(111)}/I_{(100)}$ | Intensity ratio $I_{(200)}/I_{(100)}$ |
|---|---|---|---|---|---|
| Example 1 | 2500 | 425 | 3570 | 17% | 143% |
| Example 2 | 9853 | 2280 | 15520 | 23% | 158% |
| Example 3 | 10002 | 1757 | 14689 | 18% | 147% |
| Example 4 | 10010 | 1910 | 14276 | 19% | 143% |
| Example 5 | 9935 | 1997 | 18539 | 20% | 187% |
| Comparative example 1 | 15650 | 7649 | 13700 | 49% | 88% |

From the results shown in Table 2, it was found that the intensity ratio $I_{(111)}/I_{(100)}$ of the second peak to the first peak of the compound according to each of examples 1 to 5 was smaller than the intensity ratio $I_{(111)}/I_{(100)}$ of comparative example 1 (49%). The intensity ratio $I_{(111)}/I_{(100)}$ of the compound according to each of examples 1 to 5 was, for example, more than 0% and 40% or less. In comparative example 1, the intensity of the third peak was smaller than the intensity of the first peak, whereas in each of examples 1 to 5, the intensity of the third peak was larger than the intensity of the first peak. That is, the intensity ratio $I_{(200)}/I_{(100)}$ of the third peak to the first peak in each of examples 1 to 5 was more than 100%. From such a feature of the intensity ratio, it was ascertained that the compounds of examples 1 to 5 had iodine deficiencies more than those in the compound of comparative example 1.

Further, the compounds of example 1 and comparative example 1 were subjected to XRD measurement by using an XRD apparatus with higher accuracy so as to examine in detail the positions of the fourth peak assigned to the (110) planes and the fifth peak assigned to the (220) planes.

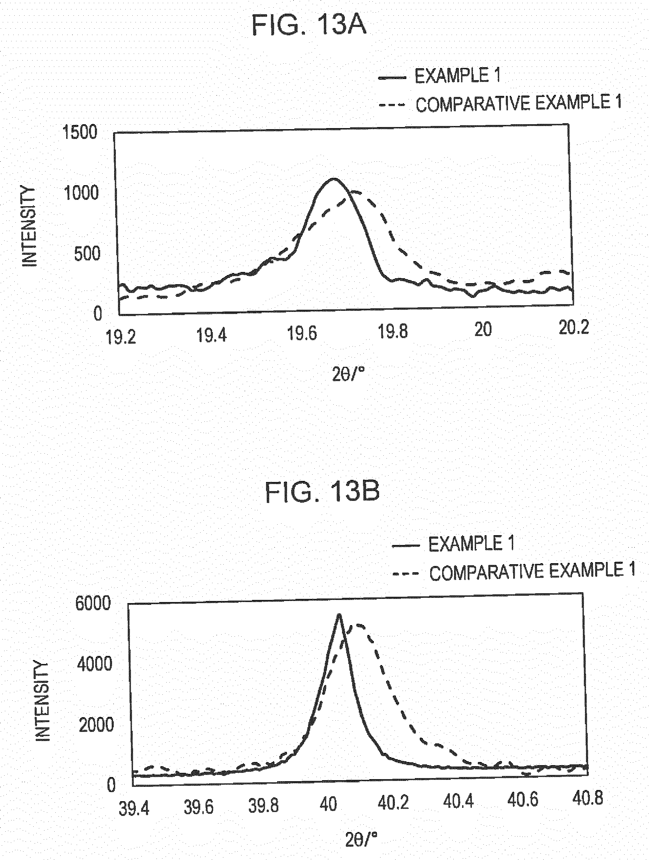

Fig. 13A and Fig. 13B are diagrams showing XRD patterns of the compounds of example 1 and comparative example 1. The solid line indicates the XRD pattern of the compound of example 1, and the broken line indicates the XRD pattern of the compound of the comparative example 1. Fig. 13A shows the diffraction intensity at a diffraction angle $2\theta$ in the range of 19.2° to 20.2°, and Fig. 13B shows the diffraction intensity at a diffraction angle $2\theta$ in the range of 39.4° to 40.8°.

As shown in Fig. 13A and Fig. 13B, the XRD pattern of example 1 has the fourth peak located at $19.65° \leq 2\theta \leq 19.71°$ and the fifth peak located at $39.98° \leq 2\theta \leq 40.10°$.

The fourth peak in example 1 was located at $2\theta = 19.68°$ and shifted from the peak ($2\theta = 19.72°$) assigned to the (110) planes in comparative example 1 to the low angle side by about 0.1°. Likewise, the fifth peak in example 1 was located at $2\theta = 40.04°$ and shifted from the peak ($2\theta = 40.11°$) assigned to the (220) planes in comparative example 1 to the low angle side by about 0.1°. This indicates that the unit cell of the $FAPbI_3$ crystal in the compound of example 1 is larger than the unit cell of the $FAPbI_3$ crystal in the compound of comparative example 1. Usually, as the unit cell of the $FAPbI_3$ crystal increases, the band gap decreases. Therefore, it was ascertained that the compound of example 1 had a band gap smaller than the band gap of the compound of comparative example 1. Fluorescence measurement and fluorescence lifetime

The compounds of examples 1 to 5 and comparative example 1 were subjected to fluorescence measurement and fluorescence lifetime measurement by using a laser of 532 nm as a light source.

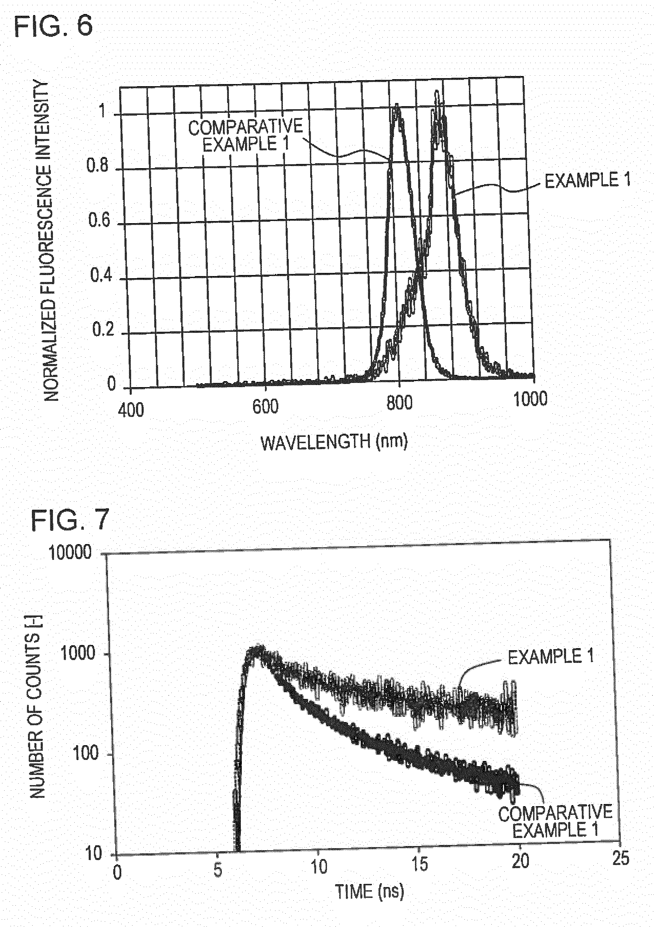

Fig. 6 is a diagram showing the measurement results of fluorescence spectra obtained by fluorescence measurement of compounds of example 1 and comparative example 1 by using the laser of 532 nm as the light source. The horizontal axis is for the wavelength and the vertical axis is for the fluorescence intensity. In this regard, the measurement results of other examples exhibited the same tendency as example 1 and, therefore, are not shown in the drawing.

As shown in Fig. 6, the fluorescence spectrum of the compound of comparative example 1 had a peak at 830 nm, whereas the fluorescence spectrum of the compound of example 1 had a peak at 890 nm. That is, it was found that the peak of the fluorescence spectrum of the compound of example 1 was located on the longer wavelength side compared with the peak of comparative example 1.

The wavelength at which the fluorescence intensity was the highest (hereafter referred to as "peak wavelength") in each of examples and comparative examples is shown in Table 3. As is clear from the results shown in Table 3, the peak wavelengths in examples 1 to 5 were 880 nm or more and 905 nm or less.

Subsequently, the fluorescence lifetime was measured at a wavelength at which the fluorescence intensity is the highest (peak wavelength). The measurement temperature was set to be 25°C.

Fig. 7 shows fluorescence decay curves of the compounds of example 1 and comparative example 1. The horizontal axis of Fig. 7 is for the time and the vertical axis is for the number of counts. Quadratic linear approximation was performed from the time at which the intensity count is the highest, and the decay rate constant of the component having the longest lifetime was calculated as the fluorescence lifetime. Regarding example 2 to example 5, the fluorescence lifetimes were calculated in the same manner. The results are shown in Table 3.

Table 3

|  | Peak wavelength of fluorescence spectrum (nm) | Fluorescence lifetime (ns) |
|---|---|---|
| Example 1 | 890 | 18.5 |
| Example 2 | 892 | 16.1 |
| Example 3 | 882 | 15.8 |
| Example 4 | 902 | 17.3 |
| Example 5 | 890 | 18.3 |
| Comparative example 1 | 833 | 5.0 |

From the results shown in Table 3, it was ascertained that the compounds of example 1 to example 5 had fluorescence lifetimes longer than the fluorescence lifetime of the compound of comparative example 1.

Absorbance measurement

The absorbance of the compound of each of examples 1 to 5 and comparative example 1 was measured, and the band gap and the absorption edge wavelength were calculated.

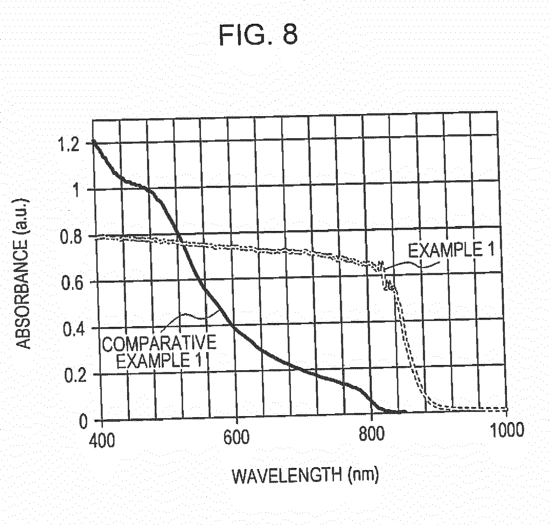

Fig. 8 is a diagram showing absorption spectra of the compounds of example 1 and comparative example 1, the horizontal axis is for the wavelength, and the vertical axis is for the absorbance. Regarding the compound of comparative example 1, the wavelength at the absorption edge corresponding to the band gap was about 830 nm and the band gap was 1.49 eV. Meanwhile, regarding the compound of example 1, the wavelength at the absorption edge was about 885 nm and the band gap was about 1.4 eV. The absorption edge wavelengths and the band gaps in example 2 to example 5 were determined in the same manner. The results are shown in Table 4.

The band gap was determined as described below. When the absorbance is assumed to be $\alpha$ and the band gap is assumed to be Eg, $\alpha^2$ is proportionate to (hv - Eg), where h denotes the Planck constant, v denotes a frequency, hv is about $1240/\lambda$, and $\lambda$ denotes an absorption wavelength. In accordance with this relationship, $\alpha^2$ was set for the vertical axis (y-axis), $1240/\lambda$ was set for the horizontal axis (x-axis), linear approximation was performed in the vicinity of the absorption edge, and the x-axis intercept thereof was taken as the band gap Eg.

Table 4

|  | Absorption edge wavelength (nm) | Band gap (eV) |
|---|---|---|
| Example 1 | 885 | 1.40 |
| Example 2 | 887 | 1.40 |
| Example 3 | 882 | 1.41 |
| Example 4 | 882 | 1.41 |
| Example 5 | 885 | 1.40 |
| Comparative example 1 | 832 | 1.49 |

From these results, it was found that the absorption edge wavelengths of the compounds of examples 1 to 5 were present on the longer wavelength side compared with the absorption edge wavelength of the compound of comparative example 1, It was ascertained that the compounds of examples 1 to 5 had band gaps of 1.4 eV or close to 1.4 eV so as to contribute to high conversion efficiency.

Each of the compounds of examples and comparative examples was subjected to a plurality of types of analysis, and the results were not always obtained from the same target position and depth of the component. For example, the target of the RBS measurement was a region with a width of 1 mm at a depth of 1 $\mu$m in the compound, The target of the XPS measurement was a region with a width of 100 $\mu$m at a depth of several nanometers in the compound. The target of the fluorescence wavelength and fluorescence lifetime measurement was a region with a width of 100 $\mu$m at a depth of 0.5 $\mu$m in the compound.

Structure of and manufacturing method for solar cell

The structure of and the manufacturing method for a solar cell by using the light absorption material according to the present embodiment will be described with reference to the drawings.

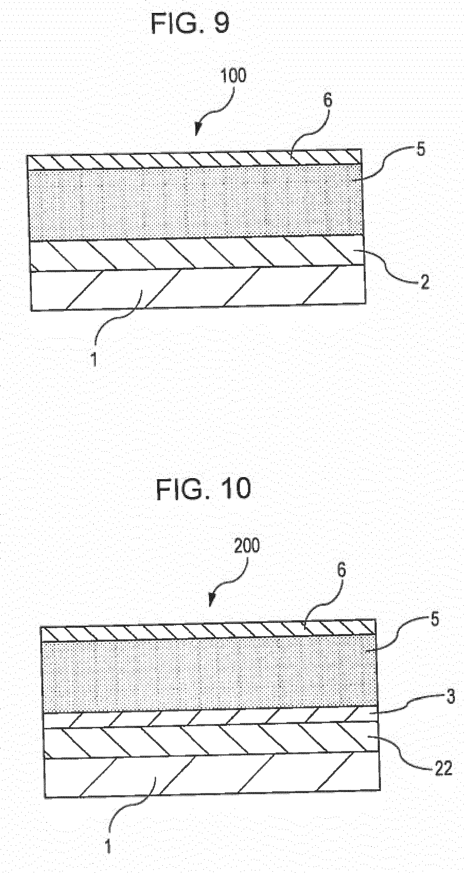

Fig. 9 is a schematic sectional view showing an example of a solar cell according to the present embodiment.

In a solar cell 100, a first electrode 2, a light absorption layer 5, and a second electrode 6 are stacked in this order on a substrate 1. The light absorption material for forming the light absorption layer 5 contains the FAPbI$_3$ compound according to the present embodiment. It is not always necessary that the solar cell 100 include the substrate 1.

Next, basic operations and advantages of the solar cell 100 will be described. In the case where the solar cell 100 is irradiated with light, the light absorption layer 5 absorbs the light so as to generate excited electrons and holes. The excited electrons move to the first electrode 2. Meanwhile, holes generated in the light absorption layer 5 move to the second electrode 6. Consequently, the solar cell 100 can output a current from the first electrode 2 serving as a negative electrode and the second electrode 6 serving as a positive electrode.

The solar cell 100 can be produced by, for example, the following method. A first electrode 2 is formed on the surface of a substrate 1 by a chemical vapor deposition method, a sputtering method, or the like. Subsequently, a light absorption layer 5 is formed on the first electrode 2. For example, the perovskite compound (FAPbI$_3$ crystal) produced by the method described above with reference to Figs. 2A to 2C may be cut so as to have a predetermined thickness, and be disposed on the first electrode so as to serve as the light absorption layer 5. Subsequently, the solar cell 100 can be produced by forming a second electrode 6 on the light absorption layer 5.

Each of constituents of the solar cell 100 will be specifically described below.

Substrate 1

The substrate 1 is an accessory constituent. The substrate 1 supports the layers of the solar cell 100. The substrate 1 can be formed of a transparent material. For example, a glass substrate or a plastic substrate (including a plastic film) can be used. In the case where the first electrode 2 has sufficient strength, it is not always necessary to dispose the substrate 1 because the layers can be supported by the first electrode 2.

First electrode 2

The first electrode 2 has electrical conductivity. The first electrode 2 does not come into ohmic contact with the light absorption layer 5. Further, the first electrode 2 has a property of blocking holes generated from the light absorption layer 5. The property of blocking holes generated from the light absorption layer 5 refers to a property of passing only electrons generated from the light absorption layer 5 and not passing holes. Materials having such a property are materials having Fermi levels lower than the lowermost energy level of the valence band of the light absorption layer 5. Specific examples of the materials include aluminum.

The first electrode 2 has a light transmitting property. For example, the light in the visible region to near-infrared region is transmitted. The first electrode 2 can be formed by using, for example, a transparent and electrically conductive metal oxide. Examples of such metal oxides include an indium-tin composite oxide, antimony-doped tin oxide, fluorine-doped tin oxide, zinc oxide doped with at least one of boron, aluminum, gallium, and indium, and composites thereof.

Alternatively, the first electrode 2 can be formed by using a material that is not transparent and providing the material with a pattern that passes light. Examples of patterns that pass light include punching-metal like patterns, in which linear, wavy line-shaped, lattice-shaped, or many fine through holes are regularly or irregularly arranged, and patterns in which negative and positive are reverse to those of the above-described patterns. In the case where the first electrode 2 has these patterns, the light can pass through the portions in which the electrode material is not present. Examples of materials that are not transparent include platinum, gold, silver, copper, aluminum, rhodium, indium, titanium, iron, nickel, tin zinc, and alloys containing any one of these. Also, carbon materials having electrical conductivity can be used.

The light transmittance of the first electrode 2 may be, for example, 50% or more, or 80% or more. The wavelength of the light to be transmitted depends on the absorption wavelength of the light absorption layer 5. The thickness of the first electrode 2 is within the range of, for example, 1 nm or more and 1,000 nm or less.

Light absorption layer 5

The light absorption material of the light absorption layer 5 contains the perovskite compound according to the present embodiment. The thickness of the light absorption layer 5 is, for example, 100 nm or more and 1,000 nm or less although the thickness depends on the magnitude of light absorption. As described above, the light absorption layer 5 may be formed by cutting a $FAPbI_3$ crystal. There is no particular limitation regarding the method for forming the light absorption layer 5. For example, formation can be performed by employing a coating method using a solution containing the perovskite compound.

Second electrode 6

The second electrode 6 has electrical conductivity. The second electrode 6 does not come into ohmic contact with the light absorption layer 5. Further, the second electrode 6 has a property of blocking electrons generated from the light absorption layer 5. The property of blocking electrons generated from the light absorption layer 5 refers to a property of passing only holes generated from the light absorption layer 5 and not passing electrons. Materials having such a property are materials having Fermi levels higher than the uppermost energy level of the conduction band of the light absorption layer 5. Specific examples of the materials include gold and carbon materials, e.g., graphene.

Fig. 10 is a schematic sectional view showing another example of the solar cell according to the present embodiment. A solar cell 200 is different from the solar cell 100 shown in Fig. 9 in that an electron transport layer is included. The constituents having the same function and configuration as those in the solar cell 100 are denoted by the same reference numerals as those in the solar cell 100 and explanations thereof may be omitted appropriately.

In the solar cell 200, a first electrode 22, an electron transport layer 3, a light absorption layer 5, and a second electrode 6 are stacked in this order on a substrate 1. It is not always necessary that the solar cell 200 include the substrate 1.

Next, basic operations and advantages of the solar cell 200 will be described. In the case where the solar cell 200 is irradiated with light, the light absorption layer 5 absorbs the light so as to generate excited electrons and holes. The excited electrons move to the first electrode 22 through the electron transport layer 3. Meanwhile, holes generated in the light absorption layer 5 move to the second electrode 6. Consequently, the solar cell 200 can output a current from the first electrode 22 serving as a negative electrode and the second electrode 6 serving as a positive electrode.

In the present embodiment, the electron transport layer 3 is disposed. Consequently, it is not necessary that the first electrode 22 has a property of blocking holes generated from the light absorption layer 5. Therefore, the width of selection of the material for forming the first electrode 22 is broadened.

The solar cell 200 according to the present embodiment can be produced in the same manner as the solar cell 100 shown in Fig. 9. The electron transport layer 3 is formed on the first electrode 22 by a sputtering method or the like.

Each of constituents of the solar cell 200 will be specifically described below.

First electrode 22

The first electrode 22 has electrical conductivity. The first electrode 22 may has the same configuration as the configuration of the first electrode 2. In the present embodiment, the electron transport layer 3 is used and, therefore, the first electrode 22 is in no need of having a property of blocking holes generated from the light absorption layer. That is, the material for forming the first electrode 22 may be a material that comes into ohmic contact with the light absorption layer.

The first electrode 22 has a light transmitting property. For example, the light in the visible region to near-infrared region is transmitted. The first electrode 22 can be formed by using, for example, a transparent and electrically conductive metal oxide. Examples of such metal oxides include an indium-tin composite oxide, antimony-doped tin oxide, fluorine-doped tin oxide, zinc oxide doped with at least one of boron, aluminum, gallium, and indium, and composites thereof.

Alternatively, a material that is not transparent can also be used as the material for forming the first electrode 22. In that case, in the same manner as the first electrode 2, the first electrode 22 is formed so as to have a pattern that passes light. Examples of electrode materials that are not transparent include platinum, gold, silver, copper, aluminum, rhodium, indium, titanium, iron, nickel, tin zinc, and alloys containing any one of these. Also, carbon materials having electrical conductivity can be used.

The light transmittance of the first electrode 22 may be, for example, 50% or more, or 80% or more. The wavelength of the light to be transmitted depends on the absorption wavelength of the light absorption layer 5. The thickness of the first electrode 22 is, for example, 1 nm or more and 1,000 nm or less.

Electron transport layer 3

The electron transport layer 3 contains a semiconductor. The electron transport layer 3 may be a semiconductor having a band gap of 3.0 eV or more. In the case where the electron transport layer 3 is formed of the semiconductor having a band gap of 3.0 eV or more, the visible light and the infrared light can be transmitted to the light absorption layer 5. Examples of semiconductors include organic and inorganic n-type semiconductors.

Examples of organic n-type semiconductors include imide compounds, quinone compounds, and fullerene and derivatives thereof. Examples of inorganic n-type semiconductors include metal element oxides and perovskite oxides. Examples of metal element oxides include oxides of Cd, Zn, In, Pb, Mo, W, Sb, Bi, Cu, Hg, Ti, Ag, Mn, Fe, V, Sn, Zr, Sr, Ga, and Cr. A more specific example is $TiO_2$. Examples of the perovskite oxides include $SrTiO_3$ and $CaTiO_3$.

The electron transport layer 3 may be formed of a material having a band gap of more than 6 eV. Examples of materials having a band gap of more than 6 eV include halides of alkali metals and alkaline earth metals, e.g., lithium fluoride and calcium fluoride, alkali metal oxides, e.g., magnesium oxide, and silicon dioxide. In this case, in order to ensure the electron transport property of the electron transport layer 3, the electron transport layer 3 is configured to become, for example, 10 nm or less.

The electron transport layer 3 may include a plurality of layers composed of materials different from each other.

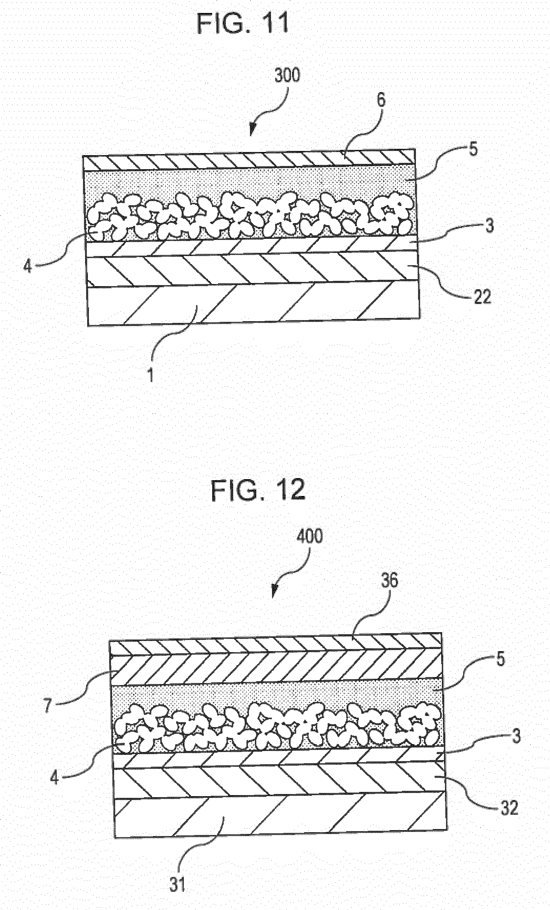

Fig. 11 is a schematic sectional view showing an example of the solar cell according to the present embodiment. A solar cell 300 is different from the solar cell 200 shown in Fig. 10 in that a porous layer is included. The constituents having the same functions and configurations as those in the solar cell 200 are denoted by the same reference numerals as those in the solar cell 200 and explanations thereof may be omitted appropriately.

In the solar cell 300, a first electrode 22, an electron transport layer 3, a porous layer 4, a light absorption layer 5, and a second electrode 6 are stacked in this order on a substrate 1. The porous layer 4 contains a porous body. The porous body includes pores. It is not always necessary that the solar cell 300 include the substrate 1.

Regarding pores in the porous layer 4, portions in contact with the light absorption layer 5 are connected up to portions in contact with the electron transport layer 3. Consequently, the pores in the porous layer 4 are filled with the material for the light absorption layer 5 and, thereby, the material can reach the surface of the electron transport layer 3. Therefore, the light absorption layer 5 and the electron transport layer 3 are in contact with each other, and giving and receiving of electrons can be directly performed.

Next, basic operations and advantages of the solar cell 300 will be described. In the case where the solar cell 300 is irradiated with light, the light absorption layer 5 absorbs the light so as to generate excited electrons and holes. The excited electrons move to the first electrode 22 through the electron transport layer 3. Meanwhile, holes generated in the light absorption layer 5 move to the second electrode 6. Consequently, the solar cell 300 can output a current from the first electrode 22 serving as a negative electrode and the second electrode 6 serving as a positive electrode.

In addition, an advantage that the light absorption layer 5 can be formed easily is obtained because the porous layer 4 is disposed on the electron transport layer 3. That is, the porous layer 4 is disposed, the material for forming the light absorption layer 5 enters the pores of the porous layer 4 and, thereby, the porous layer 4 serves as a foothold for the light absorption layer 5. Consequently, the material for forming the light absorption layer 5 is not easily repelled at or does not easily agglomerates on the surface of the porous layer 4. Therefore, the light absorption layer 5 can be formed into a uniform film.

Also, an effect of increasing the optical path length of the light that passes through the light absorption layer 5 is expected because scattering of the light occurs by the porous layer 4. It is estimated that the amounts of electrons and holes generated in the light absorption layer 5 are increased by an increase in optical path length.

The solar cell 300 can be produced in the same manner as the solar cell 200. The porous layer 4 is formed on the electron transport layer 3 by, for example, a coating method.

Porous layer 4

The porous layer 4 serves as a foundation for forming the light absorption layer 5. The porous layer 4 does not hinder light absorption of the light absorption layer 5 and movement of electrons from the light absorption layer 5 to the electron transport layer 3.

The porous layer 4 contains a porous body. Examples of porous bodies include a porous body in which insulating or semiconductor particles are connected. For example, particles of aluminum oxide or silicon oxide can be used as the insulating particles. Inorganic semiconductor particles can be used as the semiconductor particles. Regarding the inorganic semiconductor, oxides of metal elements, perovskite oxides of metal elements, sulfides of metal elements, and metal chalcogenides can be used. Examples of oxides of metal elements include oxides of Cd, Zn, In, Pb, Mo, W, Sb, Bi, Cu, Hg, Ti, Ag, Mn, Fe, V, Sn, Zr, Sr, Ga, Si, and Cr. A more specific example is $TiO_2$. Examples of perovskite oxides of metal elements include $SrTiO_3$ and $CaTiO_3$. Examples of sulfides of metal elements include CdS, ZnS, $In_2S_3$, PbS, $Mo_2S$, $WS_2$, $Sb_2S_3$, $Bi_2S_3$, $ZnCdS_2$, and $Cu_2S$. Examples of metal chalcogenides include CdSe, $In_2Se_3$, $WSe_2$, HgS, PbSe, and CdTe.

The thickness of the porous layer 4 is desirably 0.01 $\mu$m or more and 10 $\mu$m or less, and further desirably 0.1 $\mu$m or more and 1 $\mu$m or less. It is desirable that the surface roughness of the porous layer 4 be large. Specifically, the surface roughness coefficient defined as (effective area)/(projected area) is desirably 10 or more, and further desirably 100 or more. In this regard, the projected area refers to the area of a shadow generated behind an object when the object is illuminated by light from just front. The effective area refers to the actual surface area of the object. The effective area can be calculated from the volume, which is determined on the basis of the projected area and the thickness of the object, and the specific surface area and the bulk density of the material constituting the object.

Fig. 12 is a schematic sectional view showing another example of the solar cell according to the present embodiment.

A solar cell 400 is different from the solar cell 300 shown in Fig. 11 in that a hole transport layer is included. The constituents having the same functions and configurations as those in the solar cell 100 are denoted by the same reference numerals as those in the solar cell 100 and explanations thereof may be omitted appropriately.

In the solar cell 400, a first electrode 32, an electron transport layer 3, a porous layer 4, a light absorption layer 5, a hole transport layer 7, and a second electrode 36 are stacked in this order on a substrate 31. It is not always necessary that the solar cell 400 include the substrate 31.

Next, basic operations and advantages of the solar cell 400 will be described.

In the case where the solar cell 400 is irradiated with light, the light absorption layer 5 absorbs the light so as to generate excited electrons and holes. The excited electrons move to the electron transport layer 3. Meanwhile, holes generated in the light absorption layer 5 move to the hole transport layer 7. The electron transport layer 3 is connected to the first electrode 32, and the hole transport layer 7 is connected to the second electrode 36. Consequently, the solar cell 400 can output a current from the first electrode 32 serving as a negative electrode and the second electrode 36 serving as a positive electrode.

The solar cell 400 includes the hole transport layer 7 between the light absorption layer 5 and the second electrode 36. Consequently, the second electrode 36 is in no need of having a property of blocking electrons generated from the light absorption layer 5. Therefore, the width of selection of the material for forming the second electrode 36 is broadened.

Each of constituents of the solar cell 400 will be specifically described below. Explanations of the constituent common to the solar cell 300 will be omitted.

First electrode 32 and second electrode 36

As described above, the second electrode 36 is in no need of having a property of blocking electrons generated from the light absorption layer 5. That is, the material for forming the second electrode 36 may be a material that comes into ohmic contact with the light absorption layer 5. Consequently, the second electrode 36 can be formed so as to have a light transmitting property.

At least one of the first electrode 32 and the second electrode 36 has a light transmitting property and has the same configuration as the configuration of the first electrode 2 of the solar cell 100.

One of the first electrode 32 and the second electrode 36 may be in no need of having a light transmitting property. That is, it is not always necessary to use a material having a light transmitting property and to have a pattern including an opening portion for passing the light.

Substrate 31

The substrate 31 can have the same configuration as the configuration of the substrate 1 shown in Fig. 9. In the case where the second electrode 36 has a light transmitting property, the material for forming the substrate 31 may be a material not having a light transmitting property. For example, metals, ceramics, and resin materials having a low level of light transmitting property can be used as the material for forming the substrate 31.

Hole transport layer 7

The hole transport layer 7 is composed of an organic material, an inorganic semiconductor, or the like. The hole transport layer 7 may contain a plurality of layers composed of materials different from each other.

The thickness of the hole transport layer 7 is desirably 1 nm or more and 1,000 nm or less, and more desirably 10 nm or more and 50 nm or less. In this range, a sufficient hole transport property can be realized. Also low resistance can be maintained and, thereby, optical power generation can be performed with high efficiency.

Regarding a method for forming the hole transport layer 7, a coating method or a printing method can be adopted. Examples of coating methods include a doctor blade method, a bar coating method, a spraying method, a dip coating method, and a spin coating method. Examples of printing method include a screen printing method. Also, the hole transport layer 7 may be produced by mixing a plurality of materials, as necessary, and performing pressuring, firing, and the like. In the case where the material for forming the hole transport layer 7 is an organic low-molecular-weight material or an inorganic semiconductor, production can be performed by a vacuum evaporation method or the like.

The hole transport layer 7 may include a supporting electrolyte and a solvent. The supporting electrolyte and the solvent have an effect of stabilizing holes in the hole transport layer 7.

Examples of supporting electrolytes include ammonium salts and alkali metal salts. Examples of ammonium salts include tetrabutylammonium perchlorate, tetraethylammonium hexafluorophosphate, imidazolium salts, and pyridinium salts. Examples of alkali metal salts include lithium perchlorate and potassium tetrafluoroborate.

It is desirable that the solvent contained in the hole transport layer 7 has excellent ionic conductivity. Any one of aqueous solvents and organic solvents can be used, but organic solvents are preferable because a solute is more stabilized. Specific examples include heterocyclic compound solvents, e.g., tert-butylpyridine, pyridine, and n-methylpyrrolidone.

Regarding the solvent, an ionic liquid may be used alone or be used in combination with another solvent. The ionic liquid is desirable from the viewpoints of low volatility and high flame retardancy.

Examples of ionic liquids include ionic liquids of imidazolium base, e.g., 1-ethyl-3-methylimidazolium tetracyanoborate, pyridine base, alicyclic amine base, aliphatic amine base, and azonium amine base.

Examples and comparative examples

Solar cells of examples and comparative examples were produced and the element characteristics were evaluated. The methods and the results thereof will be described.

Production of solar cells of examples and comparative examples

Example 11

In example 11, a solar cell having the configuration shown in Fig. 10 was produced. The compound (FAPbI$_3$ crystal) of example 1 above was used as the light absorption layer 5.

The compound of example 1 was cut into a tabular shape by using a diamond cutter, and the surface was smoothened by using sandpaper so as to obtain a tabular sample (7 mm $\times$ 7 mm) having a thickness of 200 $\mu$m.

A substrate 1 including an ITO film serving as a first electrode 22 on the surface was prepared. A SnO$_2$ layer serving as an electron transport layer 3 was formed on the ITO film by a sputtering method. The above-described tabular test piece serving as the light absorption layer 5 was disposed on the SnO$_2$ layer. Thereafter, gold was evaporated on the surface of the sample so as to form a second electrode 6. In this manner, the solar cell of example 11 was obtained. The size (area) of the solar cell was 7 mm $\times$ 7 mm. The constituents were as described below.

Substrate 1: glass substrate 7 mm $\times$ 7 mm, thickness of 0.7 mm

First electrode 22: ITO (surface resistance of 10 $\Omega$/square)

Electron transport layer 3: SnO$_2$, thickness of 20 nm

Light absorption layer 5: compound of example 1, thickness of 200 $\mu$m

Second electrode 6: Au, thickness of 80 nm

Comparative example 4

A solar cell having the same configuration as the configuration of example 11 was produced in the same manner as comparative example 1 except that a FAPbI$_3$ film serving as the light absorption layer 5 was formed on the SnO$_2$ layer serving as the electron transport layer 3.

Measurement of IPCE

In order to evaluate the characteristics of the solar cells of example 11 and comparative example 4, the incident photon to current conversion efficiency (IPCE) was measured. A solar simulator (OTENTO-SUNV produced by Bunkoukeiki Co., Ltd.) was used for the measurement. The energy of the light source of each wavelength was set to be 5 mW/cm$^2$.

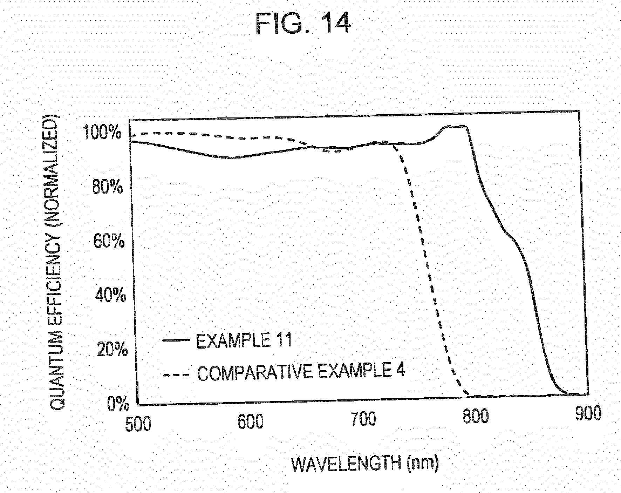

The measurement results are shown in Fig. 14. From the results shown in Fig. 14, it was found that the absorption wavelength region of the solar cell of example 11, in which the material having a high iodine deficiency rate was used for the light absorption layer 5, extended to the long wavelength side compared with the absorption wavelength region of the solar cell of comparative example 4. Regarding the solar cells of example 11 and comparative example 4, the current values under reference solar radiation (AM 1.5 G) were calculated from data of the quantum efficiencies and were 30 mA/cm$^2$ and 24 mA/cm$^2$, respectively. Therefore, it was found that the solar cell of example 11 had the current value under solar light larger than the current value of the solar cell of comparative example 4 and, therefore, could realize a high conversion efficiency.

As described above, the perovskite compound according to the present disclosure is deficient in iodine relative to Pb. For example, the element ratio of I to Pb based on the composition analysis by using X-ray photoelectron spectroscopy is 2.7 or less, or the element ratio of I to Pb based on the composition analysis by using Rutherford backscattering spectroscopy is 2.9 or less. Because of such a configuration, the perovskite compound according to the present disclosure has a small band gap compared with the perovskite compound in the related art, and has a band gap of 1.4 eV or close to 1.4 eV. Therefore, a solar cell having a high conversion efficiency can be realized by using the perovskite compound according to the present disclosure as the light absorption material of the solar cell.

The element ratio of I to Pb based on the composition analysis by using X-ray photoelectron spectroscopy of the compound is desirably 1.8 or or more. The element ratio of I to Pb based on the composition analysis by using Rutherford backscattering spectroscopy is desirably 2.0 or more. In the above-described range, the perovskite structure can be stably maintained. It is further desirable that the element ratio of I to Pb based on the composition analysis by using X-ray photoelectron spectroscopy be 2.1 or more and the element ratio of I to Pb based on the composition analysis by using Rutherford backscattering spectroscopy be 2.3 or more because the perovskite structure is more stabilized.

The light absorption material according to an embodiment of the present disclosure is suitable for use as the material for forming light absorption layers of solar cells and the like. Also, the solar cell according to an embodiment of the present disclosure is useful for optical power generation elements and optical sensors.

## Claims

1. A light absorption material comprising:

   a compound having a perovskite crystal structure represented by $ABX_3$ where the A site contains $(NH_2)_2CH^+$, the B site contains $Pb^{2+}$, and the X site contains $I^-$,
   **characterized in that**
   the X site is partially deficient in $I^-$,
   a ratio of the number of atoms of I to the number of atoms of Pb measured by an X-ray photoelectron spectroscopy is 2.7 or less, or
   a ratio of the number of atoms of I to the number of atoms of Pb measured by a Rutherford backscattering spectroscopy is 2.9 or less.

2. The light absorption material according to Claim 1, wherein the ratio of the number of atoms of I to the number of atoms of Pb measured by an X-ray photoelectron spectroscopy is 1.8 or more.

3. The light absorption material according to Claim 2, wherein the ratio of the number of atoms of I to the number of atoms of Pb measured by an X-ray photoelectron spectroscopy is 2.1 or more.

4. The light absorption material according to Claim 1, wherein the ratio of the number of atoms of I to the number of atoms of Pb measured by a Rutherford backscattering spectroscopy is 2.0 or more.

5. The light absorption material according to Claim 4, wherein the ratio of the number of atoms of I to the number of atoms of Pb measured by a Rutherford backscattering spectroscopy is 2.3 or more.

6. The light absorption material according to Claim 1, wherein the fluorescence spectrum of the compound has a peak at 880 nm or more and 905 nm or less.

7. The light absorption material according to Claim 1, wherein
   an X-ray diffraction pattern of the compound has a first peak at $13.9° \leq 2\theta \leq 14.1°$, a second peak at $23.5° \leq 2\theta \leq 24.5°$, and a third peak at $27° \leq 2\theta \leq 29°$,
   an intensity of the second peak is equal to or smaller than 40% of an intensity of the first peak, and
   an intensity of the third peak is greater than an intensity of the first peak.

8. The light absorption material according to Claim 7, wherein the X-ray diffraction pattern of the compound has a fourth peak at $19.65° \leq 2\theta \leq 19.71°$.

9. The light absorption material according to Claim 7 or 8, wherein the X-ray diffraction pattern of the compound has a fifth peak at $39.98° \leq 2\theta \leq 40.10°$.

10. A solar cell comprising:

a first electrode (2, 22, 32) having electrical conductivity;
a second electrode (6, 36) having electrical conductivity; and
a light absorption layer (5) between the first electrode (2, 22, 32) and the second electrode (6, 36), the light absorption layer comprising the light absorption material according to any of Claims 1 to 9 and converting incident light into a charge.


**Patentansprüche**

1. Lichtabsorptionsmaterial, umfassend:

eine Verbindung mit einer Perowskitkristallstruktur, die durch $ABX_3$ wiedergegeben wird, wobei die A-Position $(NH_2)_2CH^+$ enthält, die B-Position $Pb^{2+}$ enthält und die X-Position $I^-$ enthält,
**dadurch gekennzeichnet, dass**
die X-Position teilweise zu wenig $I^-$ enthält,
das Verhältnis zwischen der Anzahl von Atomen von I und der Anzahl von Atomen von Pb, das durch eine Röntgen-Photoelektronenspektroskopie gemessen wird, 2,7 oder weniger beträgt, oder
das Verhältnis zwischen der Anzahl von Atomen von I und der Anzahl von Atomen von Pb, das durch eine Rutherford-Rückstreuung-Spektroskopie gemessen wird, 2,9 oder weniger beträgt.

2. Lichtabsorptionsmaterial nach Anspruch 1, wobei das Verhältnis zwischen der Anzahl von Atomen von I und der Anzahl von Atomen von Pb, das durch eine Röntgen-Photoelektronenspektroskopie gemessen wird, 1,8 oder mehr beträgt.

3. Lichtabsorptionsmaterial nach Anspruch 2, wobei das Verhältnis zwischen der Anzahl von Atomen von I und der Anzahl von Atomen von Pb, das durch eine Röntgen-Photoelektronenspektroskopie gemessen wird, 2,1 oder mehr beträgt.

4. Lichtabsorptionsmaterial nach Anspruch 1, wobei das Verhältnis zwischen der Anzahl von Atomen von I und der Anzahl von Atomen von Pb, das durch eine Rutherford-Rückstreuung-Spektroskopie gemessen wird, 2,0 oder mehr beträgt.

5. Lichtabsorptionsmaterial nach Anspruch 4, wobei das Verhältnis zwischen der Anzahl von Atomen von I und der Anzahl von Atomen von Pb, das durch eine Rutherford-Rückstreuung-Spektroskopie gemessen wird, 2,3 oder mehr beträgt.

6. Lichtabsorptionsmaterial nach Anspruch 1, wobei das Fluoreszenzspektrum der Verbindung eine Spitze bei 880 nm oder mehr und 905 nm oder weniger aufweist.

7. Lichtabsorptionsmaterial nach Anspruch 1, wobei:

ein Röntgen-Beugungsmuster der Verbindung eine erste Spitze bei $13,9° \leq 2\theta \leq 14,1°$, eine zweite Spitze bei $23,5° \leq 2\theta \leq 24,5°$ und eine dritte Spitze bei $27° \leq 2\theta \leq 29°$ aufweist,
die Intensität der zweiten Spitze gleich oder kleiner als 40% der Intensität der ersten Spitze ist, und
die Intensität der dritten Spitze größer als die Intensität der ersten Spitze ist.

8. Lichtabsorptionsmaterial nach Anspruch 7, wobei das Röntgen-Beugungsmuster der Verbindung eine vierte Spitze bei $19,65° \leq 2\theta \leq 19,71°$ aufweist.

9. Lichtabsorptionsmaterial nach Anspruch 7 oder 8, wobei das Röntgen-Beugungsmuster der Verbindung eine fünfte Spitze bei $39,98° \leq 2\theta \leq 40,10°$ aufweist.

10. Solarzelle, die umfasst:

eine erste Elektrode (2, 22, 32), die eine elektrische Leitfähigkeit aufweist,
eine zweite Elektrode (6, 36), die eine elektrische Leitfähigkeit aufweist, und

eine Lichtabsorptionsschicht (5) zwischen der ersten Elektrode (2, 22, 32) und der zweiten Elektrode (6, 36), wobei die Lichtabsorptionsschicht das Lichtabsorptionsmaterial gemäß einem der Ansprüche 1 bis 9 umfasst und einfallendes Licht zu einer Ladung wandelt.

**Revendications**

1. Matériau d'absorption de lumière comprenant :

   un composé ayant une structure cristalline pérovskite représentée par $ABX_3$ où le site A contient $(NH_2)_2CH^+$, le site B contient $Pb^{2+}$ et le site X contient $I^-$,
   **caractérisé en ce que**
   le site X est partiellement déficient en $I^-$ ;
   un rapport du nombre d'atomes de I au nombre d'atomes de Pb mesuré par spectroscopie photoélectronique aux rayons X est inférieur ou égal à 2,7, ou
   un rapport du nombre d'atomes de I au nombre d'atomes de Pb mesuré par spectroscopie par rétrodiffusion de Rutherford est inférieur ou égal à 2,9.

2. Matériau d'absorption de lumière selon la revendication 1, dans lequel le rapport du nombre d'atomes de I au nombre d'atomes de Pb mesuré par spectroscopie photoélectronique aux rayons X est supérieur ou égal à 1,8.

3. Matériau d'absorption de lumière selon la revendication 2, dans lequel le rapport du nombre d'atomes de I au nombre d'atomes de Pb mesuré par spectroscopie photoélectronique aux rayons X est supérieur ou égal à 2,1.

4. Matériau d'absorption de lumière selon la revendication 1, dans lequel le rapport du nombre d'atomes de I au nombre d'atomes de Pb mesuré par spectroscopie par rétrodiffusion de Rutherford est supérieur ou égal à 2,0.

5. Matériau d'absorption de lumière selon la revendication 4, dans lequel le rapport du nombre d'atomes de I au nombre d'atomes de Pb mesuré par spectroscopie par rétrodiffusion de Rutherford est supérieur ou égal à 2,3.

6. Matériau d'absorption de lumière selon la revendication 1, dans lequel le spectre de fluorescence du composé présente un pic à 880 nm ou plus et 905 nm ou moins.

7. Matériau d'absorption de lumière selon la revendication 1, dans lequel
   un diffractogramme de rayons X du composé présente un premier pic à $13,9° \leq 2\theta \leq 14,1°$, un deuxième pic à $23,5° \leq 2\theta \leq 24,5°$ et un troisième pic à $27° \leq 2\theta \leq 29°$,
   une intensité du deuxième pic est inférieure ou égale à 40 % de l'intensité du premier pic, et
   une intensité du troisième pic est supérieure à une intensité du premier pic.

8. Matériau d'absorption de lumière selon la revendication 7, dans lequel le diffractogramme de rayons X du composé présente un quatrième pic à $19,65° \leq 2\theta \leq 19,71°$.

9. Matériau d'absorption de lumière selon la revendication 7 ou 8, dans lequel le diffractogramme de rayons X du composé présente un cinquième pic à $39,98° \leq 2\theta \leq 40,10°$.

10. Cellule solaire comprenant :

    une première électrode (2, 22, 32) ayant une conductivité électrique ;
    une seconde électrode (6, 36) ayant une conductivité électrique ; et
    une couche d'absorption de lumière (5) entre la première électrode (2, 22, 32) et la seconde électrode (6, 36), la couche d'absorption de lumière comprenant le matériau d'absorption de lumière selon l'une quelconque des revendications 1 à 9 et convertissant la lumière incidente en charge.

# FIG. 1

## FIG. 2A

## FIG. 2B

## FIG. 2C

## FIG. 3A

100.00 µm

## FIG. 3B

1.00 µm

FIG. 4A

FIG. 4B

FIG. 4C

FIG. 4D

## FIG. 5A

## FIG. 5B

FIG. 6

FIG. 7

FIG. 8

FIG. 9

FIG. 10

FIG. 11

FIG. 12

## FIG. 13A

EXAMPLE 1
COMPARATIVE EXAMPLE 1

## FIG. 13B

EXAMPLE 1
COMPARATIVE EXAMPLE 1

# FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JEONG-HYEOK IM.** *Nature Nanotechnology,* November 2014, vol. 9, 927-932
- **WILLIAM SHOCKLEY.** *Journal of Applied Physics,* March 1961, vol. 32 (3), 510-519
- **GILES E EPERON.** *Energy & Environmental Science,* 2014, vol. 7 (3), 982-988
- **SHUPING PANG.** *Chemistry of Materials,* January 2014, vol. 26, 1485-1491
- **MARINA R. FILIP.** *Nature Communications,* December 2014, vol. 5, 5757